(19) Europäisches Patentamt
European Patent Office
Office européen des brevets

(11) Publication number: **0 249 407**
**B1**

(12) **EUROPEAN PATENT SPECIFICATION**

(45) Date of publication of patent specification: **30.01.91**

(21) Application number: **87304983.7**

(22) Date of filing: **05.06.87**

(51) Int. Cl.$^5$: **C 07 D 263/58,**
C 07 D 235/26,
C 07 D 209/34,
C 07 D 277/68,
C 07 D 413/12, A 61 K 31/40,
A 61 K 31/33, A 61 K 31/34,
A 61 K 31/38

(54) **Antiallergy and antiinflammatory agents.**

(30) Priority: **09.06.86 JP 133470/86**

(43) Date of publication of application:
**16.12.87 Bulletin 87/51**

(45) Publication of the grant of the patent:
**30.01.91 Bulletin 91/05**

(84) Designated Contracting States:
**AT BE CH DE ES FR GB GR IT LI LU NL SE**

(56) References cited:
**EP-A-0 039 818**
**EP-A-0 051 827**
**EP-A-0 164 860**
**EP-A-0 175 551**
**WO-A-85/01289**

**PATENT ABSTRACTS OF JAPAN, vol 5, no. 128 (C-67)800r, 18th August 1981; & JP - A - 56 63972**

**The file contains technical information submitted after the application was filed and not included in this specification**

(73) Proprietor: **Pfizer Corporation**
**Calle 15 1/2 Avenida Santa Isabel**
**Colon (PA)**

(72) Inventor: **Kitaura, Yoshihiko**
**2-148 Meita-ku**
**Nagoya-shi Aichi-ken 465 (JP)**
Inventor: **Ito, Fumitaka**
**Apt. No. 206 Sakuragaoka 1-1-2 Taketoyo-cho**
**Chita-gun Aichi-ken 470-23 (JP)**
Inventor: **Stevens, Rodney William**
**Taito-Pfizer Shataku No 301 Sakuragaoka 1-1-2**
**Taketoyo-cho Chita-gun Aichi-ken 470-23 (JP)**
Inventor: **Asai, Nobuko**
**Apt. No. 404 Sakuragaoka 1-1-2**
**Taketoyo-cho Chita-gun Aichi-ken 470-23 (JP)**

(74) Representative: **Wood, David John et al**
**PFIZER LIMITED, Ramsgate Road**
**Sandwich, Kent CT13 9NJ (GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to novel heterocyclic compounds having an alkylamino group in a side chain attached to the benzene ring wherein said alkylamine group may be further substituted by an aryl or heteroaryl containing group. Those new compounds are inhibitors of both the cyclooxygenase (CO) and lipoxygenase (LO) enzymes, and are of use in the treatment or alleviation of allergic or inflammatory conditions in mammals including human.

Arachidonic acid is known to be the biological percursor of several groups of endogenous metabolies, prostaglandins including prostacyclins, thromboxances and leukotriences. The first step of the arachidonic acid metabolism is the release of esterified arachidonic acid and related unsaturated fatty acids from membrane phospholipids, *via* the action of phospholipase. Free fatty acids are then metabolized either by cyclooxygenase to produce the prostaglandins and thromboxanes or by lipoxygenase to generate hydroperoxy fatty acids which may be further converted to the leukotrienes. The prostaglandins exhibit diverse physiological effects depending upon their structure. For example, PGE and PGA inhibit gastric secretion as well as lower arterial blood pressure. The thromboxane, especially, thromboxane $A_2$ is a potent vasoconstrictor and platelet aggregatory substance. The leukotrienes are the biological source of the slow reacting substance of anaphylaxis (SRS—A), a chemical mediator in allergic bronchial asthma.

Aspirin and most other non-steroidal anti-inflammatory drugs inhibit the cyclooxygenase enzyme. Both antiinflammatory activity and analgesic activity associated with these drugs are rationalized in terms of their inhibition on the action of cyclooxygenase. The lipoxygenase inhibiting activity of one agent, AA861 (2,3,5-trimethyl-6-(12-hydroxy-5.10-cyclodecadiynyl)-1,4-benzoquinone), is reported (see, Yoshimoto et al., Biochim, et Biophys. *713*, 470—473 (1982).) CGS—5391B (C. E. Hock et al., Prostaglandins, *28*, 557—571 (1984)) has recently been known as a combination cycloxygenase and lipoxygenase inhibitor.

The determination that the compounds of this invention inhibit both cycloxygenase and lipoxygenase reflects the clinical value of these compounds in preventing proinflammatory and hypersensitivity reactions.

According to PCT Patent Application (WO 8501289) there are described and claimed a number of benzoxazolone and benzothiazolone derivatives useful for the treatment of inflammatory conditions and thrombosis.

EP—A—0164860 describes certain 2-oxindole-1-carboxamide compounds having an acyl substituent at the 3-position as analgesic and anti-inflammatory agents. EP—A—0051827 describes omega-(2-oxobenzazolinyl)-alkanoic acid derivatives as antiallergic, antiasthmatic, antiarteriosklerotic and antiinflammatory agents. EP—A—0039818 describes 2(3H)benzothiazolones as analgesic and antipyretic agents.

This invention provides novel heterocyclic compounds of this formula

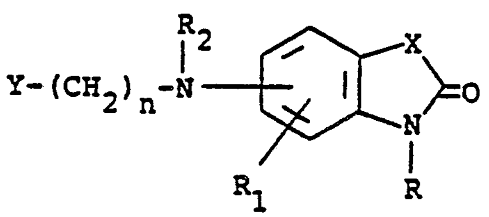

(I)

or a pharmaceutically acceptable acid addition salt thereof, wherein R is hydrogen, alkyl of one to three carbon atoms, benzyl or carboxymethyl; $R_1$ is hydrogen, fluoro, chloro, methoxy, methyl or trifluoromethyl; $R_2$ is hydrogen, alkyl of one to three carbon atoms, alkanoyl of one to three carbon atoms or benzyl; X is C=O, $CH_2$, NH or O; *n* is 1 to 7; and Y is styryl, methyl, phenylamino, phenoxy, thienyl, furyl, pyridyl, phenyl or substituted phenyl wherein the substituent is methyl, methoxy, fluoro or chloro.

The pharmaceutically acceptable acid addition salts of the compounds of the formula (I) are those formed from acids which form non-toxic acid addition salts, for example, the hydrochloride, hydrobromide, sulfate or bisulfate, phosphate or acid phosphate, acetate, citrate, fumarate, gluconate, lactate, maleate, succinate, tartrate, methanesulfonate, benzene sulfonate and toluenesulfonate, formate salts.

In formula (I) the positions of substitution by the Y containing alkylamino group and by $R_1$ other than hydrogen are not limited but are each preferably at 4-position, 5-position and 6-position of the benzo ring, both groups being more preferably in an ortho relation to each other.

A preferred group of compounds of the present invention are those wherein X is C=O and *n* is 3. Especially favored within this group is the compound where Y is phenyl and R, $R_1$ and $R_2$ are each hydrogen.

A second preferred group of compounds are those wherein X is $CH_2$ and *n* is 1 or 3. Especially preferred within this group are the compounds where Y is phenyl and R, $R_1$ and $R_2$ are each hydrogen.

A third group of favored compounds are those wherein X is NH, R is alkyl of one of three carbon atoms, $R_1$ is alkoxy of one to three carbon atoms and $R_2$ is hydrogen. Especially preferred are compounds where Y is styryl and *n* is 1, and those where Y is phenyl and *n* is 1 or 3.

A fourth group of favored compounds are those where X is O, Y is styryl, methyl, phenyl, thienyl or

substituted phenyl where said substituent is methyl, methoxy, fluoro or chloro, R is hydrogen or alkyl of one to three carbon atoms, $R_1$ is hydrogen, fluoro, chloro, methoxy or methyl, and $R_2$ is hydrogen or alkyl of one to three carbon atoms. Especially preferred are compounds where Y is methyl or phenyl, R and $R_1$ are each hydrogen and *n* is 1, 3 or 5. Also especially favored in this group are compounds where Y is phenyl, R and $R^2$ are each hydrogen, *n* is 1, 3 or 5 and $R_1$ is methyl, methoxy, fluoro or chloro substituted at the 5-position.

Especially preferred individual compounds of this invention:
6-(3-phenylpropylamino)-2-benzoxazolone;
6-(5-phenylpentylamino)-2-benzoxazolone;
6-(n-heptylamino)-2-benzoxazolone;
6-N-methyl-N'-(3-phenylpropylamino)-2-benzoxazolone;
5-Fluoro-6-(3-phenylpropylamino)-2-benzoxazolone;
5-Methyl-6-(3-phenylpropylamino)-2-benzoxazolone;
5-Methoxy-6-(3-phenylpropylamino)-2-benzoxazolone; and

The present invention also includes a pharmaceutical composition comprising a pharmaceutically acceptable carrier or diluent and a compound of formula (I), and the compounds of the formula (J) for treating an allergic or inflammatory condition in a mammal, especially man, by administering to said mammal an antiallergy or antiinflammatory effective amount of a compound of formula (I).

The compounds of formula (I) may be prepared by a number of different routes. In one embodiment, they are prepared from an amino-substituted compounds of the formula (II) according to the following reaction steps:

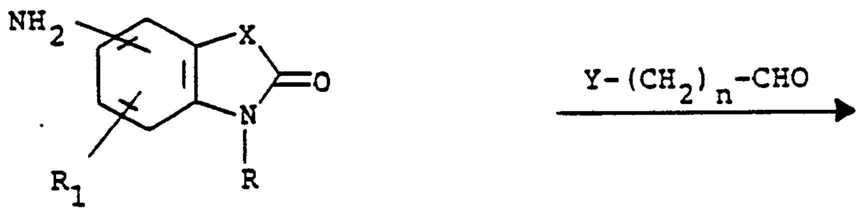


(II)

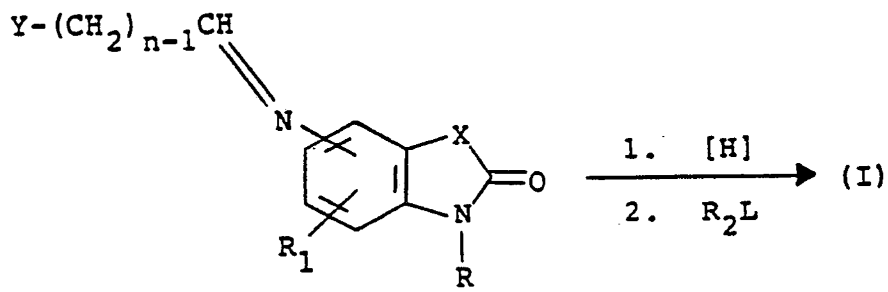


(III)

In the above formulae, X, *n*, R and $R_1$ are as previously defined, and L is a good leaving group. The first step involves the treatment of compound (II) with an aldehyde, Y—$(CH_2)_{n-1}$—CHO, in the presence of a dehydrating agent. The reaction is preferably conducted at ambient temperature. Higher temperatures up to 80°C can be employed without any significant disadvantages. Suitable solvents which do not react with the reactants and/or products are, for example, benzene, toluene and tetrahydrofuran. The preferred dehydrating agent is molecular sieves, although inorganic salts such as magnesium sulfate, potassium hydrogensulfite and sodium sulfate can also be employed. When the preferred temperature is used, the reaction is substantially complete within a few hours. On completion, the product (II) can be isolated and/or purified conventionally e.g. recrystallization or purified conventionally e.g. recrystallization or chromatographing. It is, however, more convenient not to isolate this product but to subject it (i.e., *in situ*) to reaction conditions of the second step.

The starting materials (II) and the aldehyde Y—$(CH_2)_{n-1}$—CHO are either known compounds or may be prepared by methods reported in the prior part references, see e.g., R. L. Clark and A. A. Pssolano, *J. Am. Chem. Soc., 80,* 1662 (1958).

The second step involves reduction of the C=N double bond by reaction with an appropriate hydrogen source. Reduction can be carried out in a number of ways. Compounds (III) may be reduced catalytically with hydrogen. It is normally achieved with a heterogeneous catalyst such as platinum ($PtO_2$), palladium (Pd/C) or nickel in e.g. methanol or ethanol at ambient temperature. Heating is possible but is not generally

necessary. Alternatively, the compounds may be reduced using a metal hydride. The hydride agents suitably employed in this reduction include sodium borohydride, sodium cyanoborohydride and lithium cyano borohydride. This reaction is conducted at ambient temperature, with an excess of the hydride agent in e.g. methanol or ethanol. Similar reduction using stannous chloride as a reducing agent can be carried out in methanol/aqueous hydrochloric acid. A preferred temperature for carrying out this is from 0° to 80°C. Reduction is ordinarily complete within a few hours. The product of formula (I) is isolated by standard methods known in the art. Compounds (I) wherein $R_2$ is other than hydrogen can be obtained by further reacting the reduced product with $R_2L$, e.g. alkylation. Purification can be achieved by conventional means, such as recrystallization or chromatographing.

In another embodiment, the compounds of formula (I) are prepared by the following process:

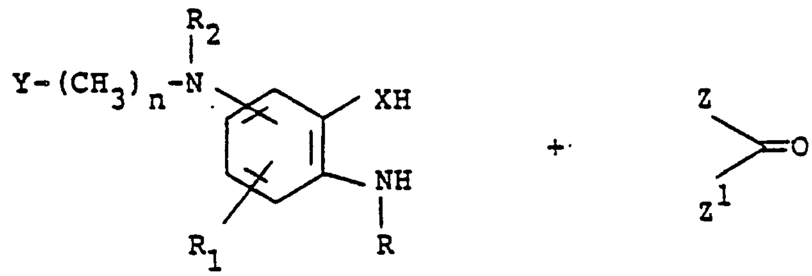

(IV) (V) (I)

In the above formulae X, n, R, $R_1$ are as previously defined and Z and Z' are both good leaving groups.

Ring forming reaction is generally performed by contacting a compound of formula (IV) with a compound of formula (V) in a reaction-inert solvent, in the presence of a base. Representative examples of compound (V) include but not limited to, phosgene, dimethyl carbonate, diethyl carbonate, urea and N, N-carbodiimidazole. A wide variety of bases can be used in the reaction and they include organic amines, alkali metal hydroxides, alkali metal carbonate, alkali metal bicarbonates, alkali metal hydrides, alkali metal alkoxides, alkaline earth metal hydroxides, alkaline earth metal carbonates, alkaline earth metal hydrides and alkaline earth metal alkaxides. Preferred basic agents are triethylamine, sodium hydroxide, sodium ethoxide, sodium hydride, calcium hydride, potassium carbonate and sodium carbonate. Suitable reaction-inert solvents include acetone, diethyl ether, tetrahydrofuran, water, $C_1$—$C_3$ alkanol, methylene chloride, N,N-dimethylformamide, and toluene. The reaction is usually carried out in the temperature range from −20°C to 100°C. Reaction times of from 30 minutes to a few hours are common. The product can be isolated and purified by conventional procedures, such as recrystallization or chromatography.

The pharmaceutically acceptable salts of the novel compounds of formula (I) are readily prepared by contacting said compound with a stoichiometric amount of an appropriate mineral or organic acid in either aqueous solution or in a suitable organic solvent. The salt may then be obtained by precipitation or by evaporation of the solvent. Among those enumerated earlier, especially preferred salt is the hydrochloride.

The compounds of formula (I) possess inhibiting activity on the action of the cyclooxygenase as well as on the action of the lipoxygenase. This activity has been demonstrated by a cell culture assay using rat peritoneal cavity resident cells which determines the effect of said compounds on the metabolism of arachidonic acid.

The ability of the compounds of formula (I) to inhibit both enzymes makes them useful for controlling the symptoms induced by the endogenous metabolites arising from arachidonic acid in a mammalian subject. The compounds are therefore valuable in the prevention and treatment of such disease states in which the accumulation of said arachidonic acid metabolite is the causative factor, e.g., allergic bronchial asthma, skin disorders, rheumatoid arthritis, osteoarthritis, and thrombosis. Thus, these compounds are of particular use in the treatment or alleviation of allergic or inflammatory conditions in a human subject.

When a compound of the formula (I) or a pharmaceutically acceptable salt thereof is to be used as either an anti-allergic agent or an anti-inflammatory agent, it can be administered to a human subject either alone, or, preferably, in combination with pharmaceutically-acceptable carriers or diluents in a pharmaceutical composition, according to standard pharmaceutical practice. A compound can be administered by a variety of conventional routes of administration including orally, parentally and by inhalation. When the compounds are administered orally, the dose range will be from about 0.1 to 20 mg/kg body weight of the subject to be treated per day, preferably from about 0.1 to 1.0 mg/kg per day in single or divided doses. If parental administration is desired, then an effective dose will be from 0.1 to 1.0 mg/kg body weight of the subject to be treated per day. In some instances it may be necessary to use dosages outside these limits, since the dosage will necessarily vary according to the age, weight and response of the individual patient as well as the severity of the patient's symptoms and the potency of the particular compound being administered.

For oral administration, the compounds of formula (I) can be administered, for exmaple, in the form of tablets, powders, lozenges, syrups or capsules, or as an aqueous solution or suspension. In the case of tablets for oral use, carriers which are commonly used include lactose and corn starch, and lubricating

agents, such as magnesium stearate, are commonly added. In the case of capsules, useful diluents are lactose and dried corn starch. When aqueous suspensions are required for oral use, the active ingredient is combined with emulsifying and suspending agents. If desired, certain sweetening and/or flavoring agents can be added. For intramuscular, intraperitoneal, subcutaneous and intravenous use, sterile solutions of the active ingredient are usually prepared, and the pH of the solutions should be suitably adjusted and buffered. For intravenous use, the total concentration of solutes should be controlled to make the preparation isotonic.

The present invention is illustrated by the following examples. Proton nuclear magnetic resonance spectra (NMR) were measured at 60 MHz unless otherwise indicated for solutions in perdeuterodimethyl sulfoxide (DMSO-$d_6$) and peak positions are expressed in parts per million (ppm) downfield from tetramethylsilane. The peak shapes are denoted as follows: s, singlet; d, doublet, t, triplet; q, quartet; m, multiplet; b, broad.

Example 1

6-(3-Phenylpropylamino)-2-benzoxazolone

To a suspension of 6-amino-2-benzoxazolone (6.00 g) and molecular sieves 4A (10.0 g) in dry benzene (100 ml) was added 3-phenylpropanal (5.25 ml), and then the mixture was stirred at room temperature for 1 hour. Methanol (150 ml) was combined with this and sodium borohydride (1.50 g) was added in portions at room temperature. The reaction mixture was filtered, the filtrate was concentrated and 5% sodium bicarbonate was added. The organic material was extracted with dichloromethane. The dichloromethane phase was separated, washed with brine. Drying and removal of solvent under reduced pressure gave a crude product which was crystallized from hot ethanol to afford 7.07 g of the title compound: mp 129°—130°C.

IR (Nujol): 3350, 3200, 1730, 1700, 1630 $cm^{-1}$.

NMR ($CDCl^3$): 1.10—2.25 (m, 2H), 2.50—4.40 (m, 3H), 6.20—7.55 (m, 8H), 8.80—9.40 (m, 1H).

Example 2

6-(3-Phenylpropylamino)-2-benzoxazolone Hydrochloride

A methanol solution (40 ml) containing 3.06 g of 6-(3-Phenylpropylamino)-2-benzoxazolone was combined with an ethereal solution saturated with gaseous hydrogen chloride (40 ml) at ice-bath temperature. The resulting solid was collected by filtration, washed with ether and dried to give the title compound (3.470 g).

m.p.: 155—158°C.

IR (Nujol): 3230, 3020, 2600, 2450, 2360, 1780, 1740, 1630 $cm^{-1}$.

NMR: 1.70—2.30 (m, 2H), 2.50—2.90 (m, 2H), 3.00—3.50 (m, 2H), 6.90—7.60 (m, 8H), 11.60—11.90 (m, 1H).

Example 3

6-(N-Methyl-N'-3-phenylpropylamino)-2-benzoxazolone Hydrochloride

To a mixed solution of 3M sulfuric acid (0.81 ml) and 40% aqueous formaldehyde (0.46 ml) was added dropwise a suspension of sodium borohydride (272 mg) and 6-(3-phenylpropylamino)-2-benzoxazolone (536 mg) at −10°C. After addition, dichloroethane (30 ml) was added and the aqueous phase was made basic by addition of 5% sodium bicarbonate solution. The organic layer was separated and washed with brine. The dried solution was evaporated at reduced pressure, and the obtained residue (479 mg) was dissolved in ethanol (10 ml). To this was added an ethereal solution saturated with gaseous hydrogen chloride (30 ml). The precipitated product was recrystallized from dichloromethane-ether to give 337 mg of the title product.

m.p.: 136—139°C.

IR (Nujol): 3300, 2650, 2580, 2500, 1780, 1630 $cm^{-1}$.

NMR: 1.50—2.20 (m, 2H), 2.40—2.90 (m, 2H), 3.15 (s, 3H), 3.30—3.80 (m, 2H), 7.00—7.90 (m, 8H).

Example 4

3-Methyl-6-(3-Phenylpropylamino)-2-benzoxazolone

By reaction of 6-amino-3-methyl-2-benzoxazolone with phenylpropanal using the procedure of Example 1, the title compound was prepared.

m.p.: 97—98°C.

IR (Nujol): 3400, 1740, 1630, 1610 $cm^{-1}$.

NMR: 1.60—2.30 (m, 2H), 2.50—3.20 (m, 4H), 3.30 (s, 3H), 3.40—3.80 (m, 1H), 6.20—6.90 (m, 3H), 7.25 (s, 5H).

Example 5

(A) 5-(3-Phenylpropylamino)-3,3-ethylenedioxy-2-oxindole

By reaction of 5-amino-3,3-ethylenedioxy-2-oxindole with 3-phenylpropanal using the procedure of Example 1, the title compound was prepared.

m.p.: 106—108°C.

IR (Nujol): 3370, 3300, 1730, 1690, 1630, 1610 $cm^{-1}$.

NMR: 1.75—2.20 (m, 2H), 2.50—3.75 (m, 5H), 4.20—4.80 (m, 4H), 6.30—6.85 (m, 3H), 7.00—7.70 (m, 6H).

(B) 5-(3-Phenylpropylamino)indole-2,3-dione hydrochloride

5-(3-Phenylpropylamino)-3,3-ethylenedioxy-2-oxindole (420 mg) was heated at reflux in a mixture of conc. hydrochloric acid (9 ml) and acetic acid (3 ml) for 1 hour. The reaction mixture was cooled to room temperature. Removal of hydrochloric acid and acetic acid under reduced pressure gave a crude product which was recrystallized from methanol-ether to afford the title compound (330 mg).

m.p.: 154—155°C.

IR (Nujol): 3220, 1730, and 1630 $cm^{-1}$.

NMR: 1.90—2.10 (m, 2H), 2.71 (t, 2H, J=8Hz), 3.24 (t, 2H, J=8Hz), 7.00 (d, 1H, J=8Hz), 7.18—7.27 (m, 5H), 7.63—7.70 (m, 2H).

Example 6

5-Benzylamino-2-oxindole

By reaction of 5-amino-2-oxindole with benzaldehyde using the procedure of Example, 1 the title compound was prepared.

m.p.: 203—205.5°C.

IR (Nujol): 3350, 1832, 1760 $cm^{-1}$.

NMR: 3.25 (s, 2H), 4.16 (d, 2H, J=6Hz), 5.72—5.85 (t, 1H), 6.39 (d, 1H, J=10Hz), 6.60—7.08 (m, 2H), 7.28 (s, 5H), 9.50 (s, 1H).

Example 7

5-(3-Phenylpropylamino)-2-oxindole

By reaction of 5-amino-2-oxindole (1.48 g) with 3-phenylpropanal using the procedure of Example 1, the title compound (1.20 g) was prepared.

m.p.: 120—122°C.

IR (Nujol): 3350, 3150, 1680, 1600 $cm^{-1}$.

NMR ($CDCl_3$): 1.40—2.20 (m 2H), 2.75 (t, 2H, J=7Hz), 3.10 (t, 2H, J=7Hz), 3.45 (s, 2H), 6.20—6.80 (m, 3H), 7.20 (s, 5H), 8.75 (br.s., 1H).

Examples 8—24

The following compounds were prepared by the method of Example 1 and Example 2 using 5-amino or 6-amino or 7-amino-2-benzoxazolone and appropriate aldehydes:

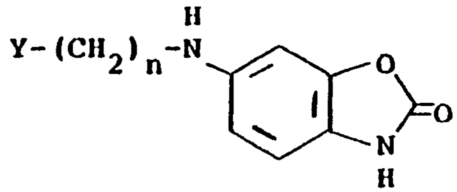

| Example No. | Y-$(CH_2)_n$-N(H)- | Form | m.p. (°C) | IR($cm^{-1}$) | NMR |
|---|---|---|---|---|---|
| 8 | 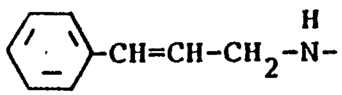 5 position | free | 139-141 | 3220, 1765, 1730 | 4.15 (d, 2H, J=4Hz), 5.60-5.90 (m, 1H), 6.38-6.50 (m, 3H), 6.78-7.03 (m, 1H), 7.30 (s, 8H), 12.19 (s, 1H) |
| 9 | 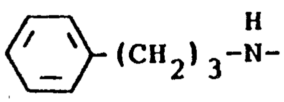 5 position | free | 77-79 | 3420, 3150, 1750, 1620, 1610 | 1.25 (br.s., 1H), 1.60-2.20 (m, 2H), 2.75 (t, 2H, J=7Hz), 3.10 (t, 2H, J=7Hz), 6.10-6.45 (m, 2H), 6.90 (d, 1H, J=9Hz), 7.25 (s, 5H), 8.50-10.50 (m, 1H) |

| Example No. | $Y-(CH_2)_n-\overset{H}{N}-$ | Form | m.p. (°C) | IR ($cm^{-1}$) | NMR |
|---|---|---|---|---|---|
| 10 | $CH_3O$-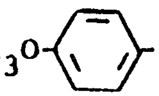-$(CH_2)_3\overset{H}{N}-$ 6 position | HCl | 248-249 | 3250, 2700, 1770, 1745 | 1.85-2.00 (m, 2H), 2.61 (t, 2H, J=7.3Hz), 3.19-3.25 (m, 2H), 3.72 (s, 3H), 6.85 (d, 2H, J=8.8Hz), 7.11 (d, 2H, J=8.8Hz), 7.18 (d, 1H, J=8.1Hz), 7.29 (d, 1H, J=8.1Hz), 7.47 (s, 1H), 11.93 (s, 1H) |
| 11 | 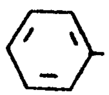-$(CH_3)_5-\overset{H}{N}-$ 5 position | free | 113-114 | 3300, 1750, 1630 | 1.20-2.00 (m, 6H), 2.40-2.80 (m, 2H), 2.85-3.30 (m, 2H), 3.40-3.90 (m, 1H), 6.10-6.50 (m, 2H), 6.95 (d, 1H, J=7Hz), 7.20 (s, 5H), 9.00-9.90 (m, 1H) |
| 12 | 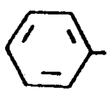-$(CH_2)_5-\overset{H}{N}-$ 6 position | free | 115-116 | 3420, 3200, 1750, 1635 | 1.00-2.10 (m, 6H), 2.30-3.90 (m, 5H), 6.20-7.70 (m, 8H), 8.50-9.60 (m, 1H) |

| Example No. | $Y-(CH_2)_n-\overset{H}{N}-$ | Form | m.p. (°C) | IR($cm^{-1}$) | NMR |
|---|---|---|---|---|---|
| 13 | 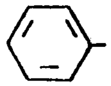 $-(CH_2)_5-\overset{H}{N}-$ 6 position | HCl | 153-155 | 3230, 3210, 2650, 2600, 2520, 2430, 2370, 1780, 1740 1630 | 1.10-2.10 (m, 6H), 2.40-2.80 (m, 2H), 3.00-3.48 (m, 2H), 7.00-7.60 (m, 8H), 11.70-12.00 |
| 13a | 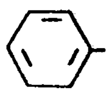 $-(CH_2)_7-\overset{H}{N}-$ 6 position | free | 102-103 | Nujol 3100, 2950, 2850, 1750 | at 270MHz 1.36-1.37 (m, 6H), 1.57-1.65 (m, 4H), 2.60 (m, 3H), 3.06 (m, 3H), 3.56 (br.s, 1H), 6.37 (dd, 1H, J=8.8, 2.2Hz), 6.50 (d, 1H, J=2.2Hz), 6.83 (d, 1H, J=8.8Hz), 7.14-7.30 (m, 5H), 8.21 (br.s., 1H) |
| 14 | 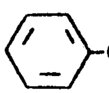 $-CH_2-\overset{H}{N}-$ 6 position | free | 168-170 | 3350, 1830, 1760, 1720 | 4.26 (d, 2H, J=6Hz), 5.99-6.18 (t, 1H), 6.34-6.86 (m, 3H), 7.32 (s, 5H), 10.80-11.0 (m, 1H) |
| 15 | 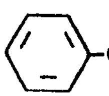 $-CH_2-\overset{H}{N}-$ 6 position | HCl | 185-186 | 3150, 1828, 1770 (KBr) | 4.42 (s, 2H), 7.05 (s, 1H), 7.17 7.69 (m, 7H), 8.08 (s, 1H), 11.58 (s, 1H) |

| Example No. | $Y-(CH_2)_n-\overset{H}{N}-$ | Form | m.p. (°C) | IR ($cm^{-1}$) | NMR |
|---|---|---|---|---|---|
| 16 | 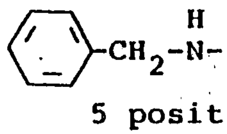 5 position | free | 141-142 | 3450, 3200, 1760, 1740, 1620 | 4.25 (d, 2H, J=6Hz), 5.70-6.10 (m, 1H), 6.10-6.40 (m, 3H), 6.85 (d, 1H, J=9Hz), 7.30 (s, 5H), 10.80-11.20 (m, 1H) |
| 17 | 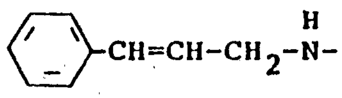 6 position | free | 164-165.5 | 3300, 1720, 1690 | 3.76-3.94 (t, 2H), 5.68 5.88 (m, 2H), 6.35-6.89 (m, 4H), 7.33 (s, 5H), 11.00 (s, 1H) |
| 18 | 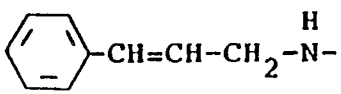 6 position | HCl | 166-168 | 3220, 1862, 1765, 1730 | 4.05 (d, 2H, J=6Hz), 6.20-6.47 (m, 2H), 6.64 (s, 1H), 6.90-7.52 (s, 8H), 8.00 (s, 1H), 11.67 (s, 1H) |

| Example No. | $Y-(CH_2)_n-\overset{N}{N}-$ | Form | m.p. (°C) | IR ($cm^{-1}$) | NMR |
|---|---|---|---|---|---|
| 19 | 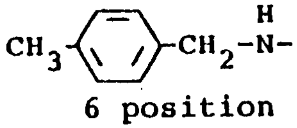 6 position | free | 151 dec. | 3410, 1740, 1640, (Nujol) | at 270MHz, 2.35 (s, 3H), 4.0 (s, 1H), 4.26 (d, 2H, J=5.1Hz), 6.42 (dd, 1H, J=8.8, 2.2Hz), 6.54 (d, 1H, J=2.2Hz), 6.84 (d, 1H, J=8.8Hz), 7.14-7.30 (m, 4H). |
| 20 | $CH_3-(CH_2)_6-\overset{H}{N}-$ 6 position | free | 101-103 | 3420, 3200, 1760, 1640, 1620 (Nujol) | 0.65-2.00 (m, 13H), 2.80-3.30 (m, 2H), 3.40-3.80 (m, 1H), 6.20-6.65 (m, 2H), 6.85 (d, 1H, J=8Hz), 8.50-9.20 (m, 1H) |
| 21 | 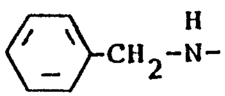 7 position | free | 193.5-196 | 3200, 1760, 1650 (KBr) | 3.42 (br.s., 1H), 4.42 (d, 2H, J=7Hz), 6.25-6.38 (m, 2H), 6.7-7.0 (m, 1H), 7.34 (s, 5H), 11.38 (br.s, 1H) |
| 22 | 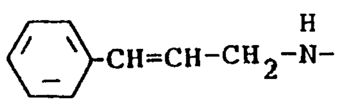 7 position | free | 173.5-175 | 3320, 1750, 1640 | 4.0 (m, 2H), 5.92 (m, 1H), 6.25-6.60 (m, 4H), 6.86 (d, 1H, J=8Hz), 7.34 (s, 5H), 11.25 (br.s., 1H) |

| Example No. | Y-(CH₂)ₙ-N- (N) | Form | m.p. (°C) | IR ($cm^{-1}$) | NMR |
|---|---|---|---|---|---|
| 23 | 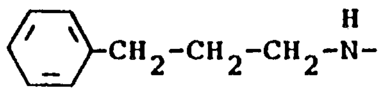 7 position | HCl | 181-183 | 3450, 1800, 1770 | 1.94 (m, 2H), 2.72 (t, 2H, J=7Hz), 3.22 (t, 2H, J=7Hz), 5.18 (br.s., 2H), 6.4-6.6 (m, 2H), 6.92 (d, 1H, J=9Hz), 7.25 (s, 5H), 11.45 (br.s, 1H) |
| 24 | 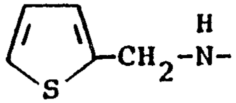 6 position | free | 166-168 | 3380, 1835, 1750, 1716 | 4.42 (d, 2H, J=6Hz), 5.90-6.10 (t, 1H), 6.34-7.00 (m, 5H), 7.29 (dd, 1H), 10.93 (br.s., 1H) |
| 24a | 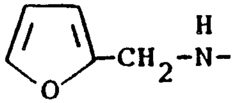 6 position | free | 142-144 | 3350, 3000, 1750, 1710 | at 270MHz in $CDCl_3$ 4.40 (s, 1H), 4.30 (s, 2H), 6.25 (s, 1H), 6.32 (s, 1H), 6.47 (d, 1H, J=8.1Hz), 6.61 (s, 1H), 6.88 (d, 1H, J=8.1Hz), 7.37 (s, 1H), 9.18 (s, 1H) |

| Example No. | Y-$(CH_2)_n$-N- | Form | m.p. (°C) | IR($cm^{-1}$) | NMR |
|---|---|---|---|---|---|
| 24b | 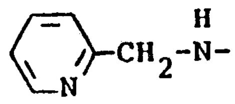 6 position | free | 164-167 | Nujol 3400, 1780 1710, 1640 | at 270MHz in $CDCl_3$ 4.43 (d, 2H, J=5Hz), 4.85 (s, 1H), 6.52 (dd, 1H, J=8.1, 2.2Hz), 6.59 (d, 1H, J=2.2Hz), 6.84 (d, 1H, J=8.1Hz), 7.19-7.34 (m, 2H), 7.65-7.70 (m, 1H), 8.59 (d, 1H, J=4.4Hz) |
| 24c | 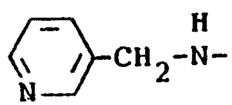 6 position | 2HCl | 205(dec) | Nujol 1760, 1630 | at 270MHz 4.51 (s, 2H), 6.54 (d, 1H, J=8.8Hz), 6.74 (s, 1H), 6.84 (d, 1H, J=8.1Hz), 7.98-8.03 (m, 1H), 8.40 (d, 1H, J=6.6Hz), 8.80 (d, 1H, J=5.1Hz), 8.88 (s, 1H), 11.30 (s, 1H) |
| 24d | 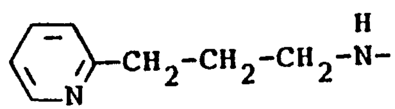 6 position | free | 170(dec) | Nujol 3370, 2650 1760, 1630 | at 270MHz 1.87-1.98 (m, 2H), 2.75-2.85 (m, 2H), 2.96-3.44 (m, 2H), 5.56 (d, 1H, J=5.9Hz), 6.35 (dd, 1H, J=8.1, 2.2Hz), 6.51 (d, 1H, J=2.2Hz), 6.79 (d, 1H, J=8.1Hz), 7.17-7.2 7.28 (m, 2H), 7.66-7.72 (m, 1H), 8.48 (d, 1H, J=5.1Hz), 11.02 (br.s, 1H) |

Examples 25—27

Reaction of 6-amino-5-methoxy-1-methyl-2-benzimidazolone with appropriate aldehydes, according to the procedure of Example 1, afforded the following compounds.

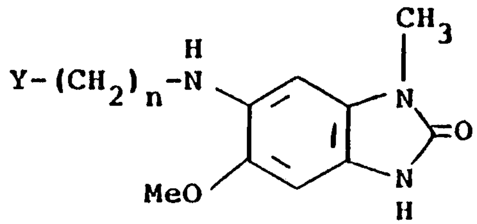

| Example No. | $Y-(CH_2)_n-\overset{H}{N}-$ | m.p. (°C) | IR(KBr) $cm^{-1}$ | NMR ppm |
|---|---|---|---|---|
| 25 | 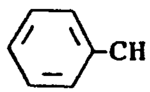 $-CH_2-\overset{H}{N}-$ | 219-222 | 3100, 1685 | 3.13 (s, 3H), 3.77 (s, 3H), 4.32 (d, 2H, J=6Hz), 5.01 (t, 1H, J=6Hz), 6.32 (s, 1H), 6.57 (s, 1H), 7.2-7.5 (m, 5H), 10.29 (s, 1H) |
| 26 | 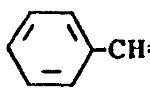 $-CH=CH-CH_2-\overset{H}{N}-$ | 213.5-215 | 3000, 1680 | 3.2 (s, 3H), 3.78 (s, 3H), 3.92 (m, 2H), 4.72 (t, 1H, J=3Hz), 6.39 (d, 1H, J=16Hz), 6.42 (s, 1H), 6.58 (s, 1H), 6.61 (d, 1H, J=16Hz), 7.20-7.4 (m, 5H), 10.30 (br.s., 1H) |
| 27 | 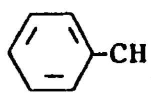 $-CH_2-CH_2-CH_2-\overset{H}{N}-$ | 182.5-184 | 3150, 1710 | 1.90 (m, 2H), 2.69 (t, 2H, J=6Hz), 3.09 (q, 2H, J=6Hz), 3.18 (s, 3H), 3.73 (s, 3H), 4.41 (t, 1H, J=3Hz), 6.26 (s, 1H), 6.55 (s, 1H), 7.14-7.30 (m, 5H), 10.26 (s, 1H) |

Examples 28—31

Reaction of 5-substituted-6-amino-2-benzoxazolone with appropriate aldehydes, according to the procedure of Example 1, afforded the following compounds.

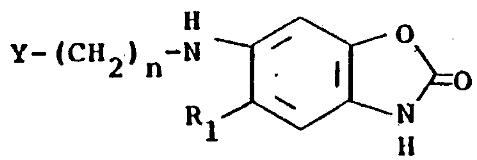

| Example No. | $Y-(CH_2)_n-\overset{H}{N}-$ | $R_1$ | m.p. (°C) | IR (Nujol) $cm^{-1}$ | NMR (ppm) |
|---|---|---|---|---|---|
| 28 | 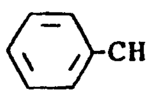$-CH_2-CH_2-CH_2-\overset{H}{N}-$ | $CH_3O-$ | 132-133 | 3480, 3200, 1790, 1730, 1635, 1600 | 1.70 (m, 2H), 2.55-3.35 (m, 4H), 3.83 (s, 3H), 4.00-4.35 (m, 1H), 6.50 (s, 1H), 6.60 (s, 1H), 7.24 (s, 5H), 9.30-9.70 (m, 1H) |

| Example No. | $Y-(CH_2)_n-\overset{H}{N}-$ | $R_1$ | m.p. (°C) | IR(Nujol) $cm^{-1}$ | NMR (ppm) |
|---|---|---|---|---|---|
| 28a | 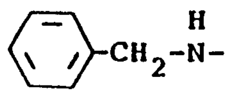 | $CH_3O-$ | 177-179 | 3450, 3150, 3100, 1760, 1640 | at 270MHz 3.80 (s, 3H), 4.30 (d, 2H, J=5.9Hz), 5.44 (t, 1Hz, J=5.9Hz, 6.43 (s, 1H), 6.63 (s, 1H), 7.18-7.37 (m, 5H), 11.05 (br.s, 1H) |
| 29 | 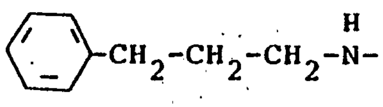 | F- | 150-152 | 3400, 3200, 1760, 1730, 1645, 1620 | 1.70-2.25 (m, 2H), 2.50-3.35 (m, 4H), 3.85-4.40 (m, 1H), 6.50 (d, 1H), J=7Hz), 6.72 (d, 1H, J=10Hz), 7.20 (s, 5H) 10.50-11.10 (m, 1H) |
| 30 | 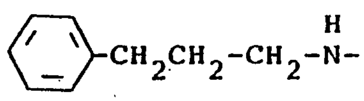 | $CH_3-$ | 135-137 | 3400, 1750, 1640, 1620 | 1.70-2.45 (m, 2H), 2.10 (s, 3H), 2.60-3.55 (m, 4H), 6.50 (s, 1H), 6.75 (s, 1H), 7.25 (s, 5H) |

| Example No. | $Y-(CH_2)_n-\overset{H}{N}-$ | $R_1$ | m.p. (°C) | IR (Nujol) $cm^{-1}$ | NMR (ppm) |
|---|---|---|---|---|---|
| 31 | 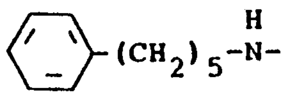 | Cl- | 133-135 | 3450, 3150, 1760, 1640, 1610 | 1.50-4.70 (m, 7H), 6.50 (s, 1H), 6.90 (s, 1H), 7.30 (s, 5H), 10.70-11.30 (m, 1H) |
| 32 | 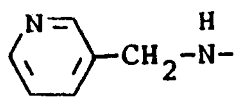 | $CH_3$- | 190-193 | 3450, 1750, 1645, 1590, 1580 | at 270MHz 2.16 (s, 3H), 4.37 (d, 2H, J=5.9Hz), 5.53-5.58 (m, 1H), 6.42 (s, 1H), 6.76 (s, 1H), 7.30-7.74 (m, 1H), 7.75 (d, 1H, J=8.1Hz), 8.43 (d, 1H, J=2.7Hz), 8.59 (s, 1H), 10.95 (br.s, 1H) |

"Nujol" is a Registered Trade Mark.

**Claims for the Contracting States: BE CH DE FR GB IT LI LU NL SE**

1. A compound of the formula:

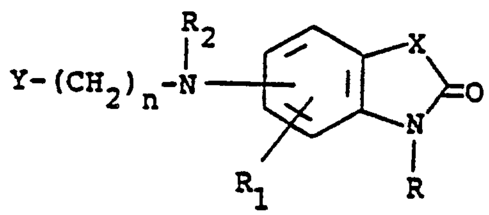

--- (I)

or a pharmaceutically acceptable acid addition salt thereof, wherein R is hydrogen, alkyl having one to three carbon atoms, benzyl or carboxymethyl; $R_1$ is hydrogen, fluoro, chloro, methoxy, methyl or trifluoromethyl; $R_2$ is hydrogen, alkyl having one to three carbon atoms, alkanoyl having one to three carbon atoms or benzyl; X is C=O, $CH_2$, NH or O; *n* is an integer of 1 to 7; and Y is styryl, methyl, phenylamino, phenoxy, thienyl, furyl, pyridyl, phenyl or substituted phenyl wherein said substituent is methyl, methoxy, fluoro or chloro.

2. A compound of claim 1, wherein X is C=O and *n* is 3.

3. A compound of claim 2, wherein Y is phenyl and R, $R_1$ and $R_2$ are each hydrogen.

4. A compound of claim 1, wherein X is $CH_2$ and *n* is 1 or 3.

5. A compound of claim 4, wherein Y is phenyl and R, $R_1$ and $R_2$ are each hydrogen.

6. A compound of claim 1, wherein X is NH, R is alkyl having one to three carbon atoms, $R_1$ is alkoxy having from one to three carbon atoms and $R_2$ is hydrogen.

7. A compound of claim 6, wherein *n* is 1 and Y is styryl.

8. A compound of claim 6, wherein *n* is 1 or 3 and Y is phenyl.

9. A compound of claim 1, wherein X is O, Y is styryl, methyl, phenyl, thienyl or substituted phenyl wherein said substituent is methyl, methoxy, fluoro or chloro; R is hydrogen or alkyl having one to three carbon atoms; $R_1$ is hydrogen, fluoro, chloro, methoxy or methyl; and $R_2$ is hydrogen or alkyl having one to three carbon atoms.

10. A compound of claim 9, wherein Y is methyl or phenyl, R and $R_1$ are each hydrogen and *n* is 1, 3 or 5.

11. A compound of claim 9, wherein Y is phenyl, R and $R_2$ are each hydrogen, *n* is 1, 3 or 5 and $R_1$ is methyl, methoxy, fluoro or chloro substituted at the 5-position.

12. A pharmaceutical composition for the treatment of allergic or inflammatory conditions in a mammal, said composition comprising an effective amount of a compound of the formula (I) as claimed in any one of the preceding claims, or a pharmaceutically acceptable acid addition salt thereof, and a pharmaceutically acceptable diluent or carrier.

13. A compound of the formula (I) as claimed in any one of claims 1 to 11, or a pharmaceutically acceptable acid addition salt thereof, for use as a medicament.

14. The use of a compound of the formula (I) as claimed in any one of claims 1 to 11, or of a pharmaceutically acceptable acid addition salt thereof, for the manufacture of a medicament for treating allergic or inflammatory conditions.

15. A compound of the formula:

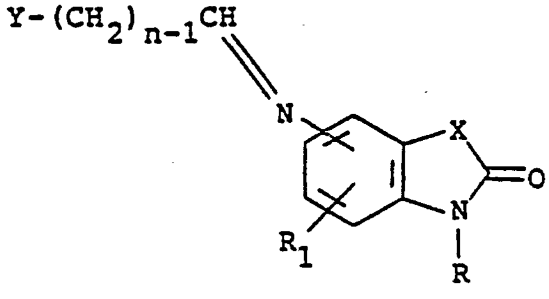

--- (III)

where R, $R_1$, X, Y and n are as defined in claim 1.

**Claims for the Contracting States: AT ES**

1. A process for preparing a compound of the formula:

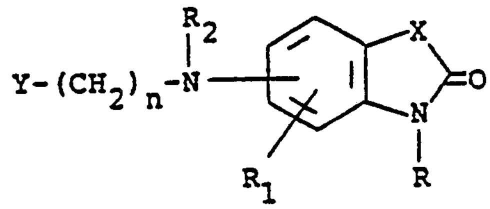

--- (I)

or a pharmaceutically acceptable acid addition salt thereof, wherein R is hydrogen, alkyl having one to three carbon atoms, benzyl or carboxymethyl; $R_1$ is hydrogen, fluoro, chloro, methoxy, methyl or trifluoromethyl; $R_2$ is hydrogen, alkyl of one to three carbon atoms, alkanoyl having one to three carbon atoms or benzyl; X is C=O, $CH_2$, NH or O; *n* is an integer of 1 to 7; and Y is styryl, methyl, phenylamino, phenoxy, thienyl, furyl, pyridyl, phenyl or substituted phenyl wherein said substituent is methyl, methoxy, fluoro or chloro; characterized by reducing a compound of the formula:

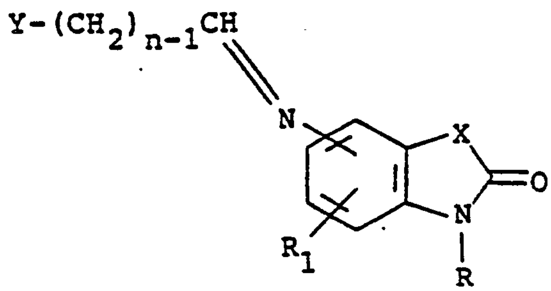

--- (III)

where Y, n, R, $R_1$ and X are as defined above, followed by, if desired, alkylating or acylating a compound of the formula (I) in which $R_2$ is hydrogen so as to produce a compound of the formula (I) in which $R_2$ is alkyl of 1 to 3 carbon atoms, benzyl or alkanoyl of 1 to 3 carbon atoms, and, optionally, converting the product of the formula (I) into a pharmaceutically acceptable acid addition salt by reaction with a suitable acid.

2. A process of claim 1, wherein the reducing agent is sodium borohydride.

3. A process of claim 1 or 2, wherein X is C=O and *n* is 3.

4. The process of claim 3, wherein Y is phenyl and R, $R_1$ and $R_2$ are each hydrogen.

5. A process of claim 1 or 2, wherein X is $CH_2$ and *n* is 1 or 3.

6. A process of claim 5, wherein Y is phenyl and R, $R_1$ and $R_2$ are each hydrogen.

7. A process of claim 1 or 2, wherein X is NH, R is alkyl having one to three carbon atoms, $R_1$ is alkoxy having from one to three carbon atoms and $R_2$ is hydrogen.

8. A process of claim 7, wherein *n* is 1 and Y is styryl.

9. A process of claim 7, wherein *n* is 1 or 3 and Y is phenyl.

10. A process of claim 1 or 2, wherein X is O, Y is styryl, methyl, phenyl, thienyl or substituted phenyl wherein said substituent is methyl, methoxy, fluoro or chloro; R is hydrogen or alkyl having one to three carbon atoms; $R_1$ is hydrogen, fluoro, chloro, methoxy or methyl; and $R_2$ is hydrogen or alkyl having one to three carbon atoms.

11. A process of claim 10, wherein Y is methyl or phenyl, R and $R_1$ are each hydrogen and *n* is 1, 3 or 5.

12. A process of claim 10, wherein Y is phenyl, R and $R_2$ are each hydrogen, *n* is 1, 3 or 5 and $R_1$ is methyl, methoxy, fluoro or chloro substituted at the 5-position.

13. A process for preparing a pharmaceutical composition characterized by mixing a compound of the formula (I) as defined in claim 1, or a pharmaceutically acceptable acid addition salt thereof, with a pharmaceutically acceptable diluent or carrier.

**Claims for the Contracting State: GR**

1. A process for preparing a compound of the formula:

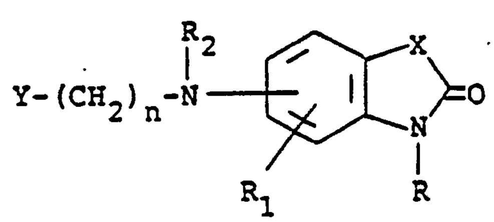

--- (I)

or a pharmaceutically acceptable acid addition salt thereof, where R is hydrogen, alkyl of one to three carbon atoms, benzyl or carboxymethyl; $R_1$ is hydrogen, fluoro, chloro, methoxy, methyl or trifluoromethyl; $R_2$ is hydrogen, alkyl of one to three carbon atoms, alkanoyl of one to three carbon atoms or benzyl; X is C=O, $CH_2$, NH or O; *n* is an integer of 1 to 7; and Y is styryl, methyl, phenylamino, phenoxy, thienyl, furyl, pyridyl, phenyl or substituted phenyl wherein said substituent is methyl, methoxy, fluoro or chloro; characterized by reducing a compound of the formula:

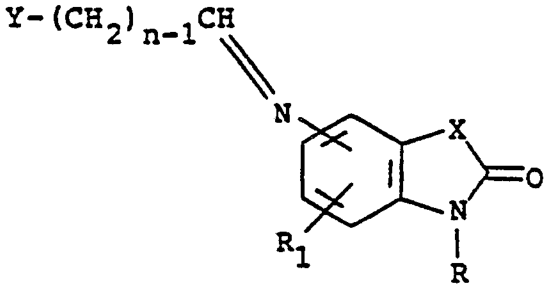

--- (III)

where Y, n, R, $R_1$ and X are as defined above, followed by, if desired, alkylating or acylating a compound of the formula (I) in which $R_2$ is hydrogen so as to produce a compound of the formula (I) in which $R_2$ is alkyl of 1 to 3 carbon atoms, benzyl or alkanoyl of 1 to 3 carbon atoms, and, optionally, converting the product of the formula (I) into a pharmaceutically acceptable acid addition salt by reaction with a suitable acid.

2. A process of claim 1, wherein the reducing agent is sodium borohydride.

3. A process of claim 1, wherein X is C=O and *n* is 3.

4. A process of claim 3, wherein Y is phenyl and R, $R_1$ and $R_2$ are each hydrogen.

5. A process of claim 1 or 2, wherein X is $CH_2$ and *n* is 1 or 3.

6. A process of claim 5, wherein Y is phenyl and R, $R_1$ and $R_2$ are each hydrogen.

7. A process of claim 1 or 2, wherein X is NH, R is alkyl having one to three carbon atoms, $R_1$ is alkoxy having from one to three carbon atoms and $R_2$ is hydrogen.

8. A process of claim 7, wherein *n* is 1 and Y is styryl.

9. A process of claim 7, wherein *n* is 1 or 3 and Y is phenyl.

10. A process of claim 1 or 2, wherein X is O, Y is styryl, methyl, phenyl, thienyl or substituted phenyl wherein said substituent is methyl, methoxy, fluoro or chloro; R is hydrogen or alkyl having one to three carbon atoms; $R_1$ is hydrogen, fluoro, chloro, methoxy or methyl; and $R_2$ is hydrogen or alkyl having one to three carbon atoms.

11. A process of claim 10, wherein Y is methyl or phenyl, R and $R_1$ are each hydrogen and *n* is 1, 3 or 5.

12. A process of claim 10, wherein Y is phenyl, R and $R_2$ are each hydrogen, *n* is 1, 3 or 5 and $R_1$ is methyl, methoxy, fluoro or chloro substituted at the 5-position.

13. A process for preparing a pharmaceutical composition characterized by mixing a compound of the formula (I) as defined in claim 1, or a pharmaceutically acceptable acid addition salt thereof, with a pharmaceutically acceptable diluent or carrier.

14. A compound of the formula:

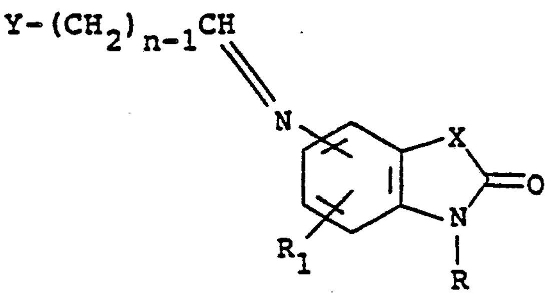

--- (III)

where Y, n, R, $R_1$ and X are as defined in claim 1.

**Patentansprüche für die Vertragsstaaten: BE CH DE FR GB IT LI LU NL SE**

1. Verbindung der Formel

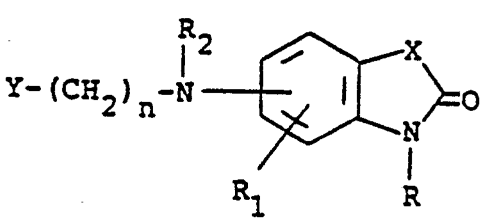

--- (I)

oder ein pharmazeutisch annehmbares Säureadditionssalz derselben, worin R Wasserstoff, Alkyl mit einem bis drei Kohlenstoffatomen, Benzyl oder Carboxymethyl ist, $R_1$ Wasserstoff, Fluor, Chlor, Methoxy, Methyl oder Trifluormethyl ist, $R_2$ Wasserstoff, Alkyl mit einem bis drei Kohlenstoffatomen, Alkanoyl mit einem bis drei Kohlenstoffatomen oder Benzyl ist, X C=O, $CH_2$, NH oder 0 ist, *n* eine ganze Zahl von 0 bis 7 ist und Y Styryl, Methyl, Phenylamino, Phenoxy, Thienyl, Furyl, Pyridyl, Phenyl oder substituiertes Phenyl ist, wobei dieser Substituent Methyl, Methoxy, Fluor oder Chlor ist.

2. Verbindung gemäß Anspruch 1, worin X C=O ist und *n* gleich 3 ist.

3. Verbindung gemäß Anspruch 2, worin Y Phenyl ist und R, $R_1$ und $R_2$ jeweils Wasserstoff sind.

4. Verbindung gemäß Anspruch 1, worin X $CH_2$ ist und *n* gleich 1 oder 3 ist.

5. Verbindung gemäß Anspruch 4, worin Y Phenyl ist und R, $R_1$ und $R_2$ jeweils Wasserstoff sind.

6. Verbindung gemäß Anspruch 1, worin X NH ist, R Alkyl mit einem bis drei Kohlenstoffatomen ist, $R_1$ Alkoxy mit einem bis drei Kohlenstoffatomen ist und $R_2$ Wasserstoff ist.

7. Verbindung gemäß Anspruch 6, worin *n* gleich 1 ist und Y Styryl ist.

8. Verbindung gemäß Anspruch 6, worin *n* gleich 1 oder 3 ist und Y Phenyl ist.

9. Verbindung gemäß Anspruch 1, worin X O ist, Y Styryl, Methyl, Phenyl, Thienyl oder substituiertes Phenyl ist, wobei dieser Substituent Methyl, Methoxy, Fluor oder Chlor ist, R Wasserstoff oder Alkyl mit einem bis drei Kohlenstoffatomen ist, $R_1$ Wasserstoff, Fluor, Chlor, Methoxy oder Methyl ist und $R_2$ Wasserstoff oder Alkyl mit einem bis drei Kohlenstoffatomen ist.

10. Verbindung gemäß Anspruch 9, worin Y Methyl oder Phenyl ist, R und $R_1$ jeweils Wasserstoff sind und *n* gleich 1, 3 oder 5 ist.

11. Verbindung gemäß Anspruch 9, worin Y Phenyl ist, R und $R_2$ jeweils Wasserstoff sind, *n* gleich 1, 3 oder 5 ist und $R_1$ Methyl, Methoxy, Fluor oder Chlor, substituiert in der 5-Position, ist.

12. Pharmazeutische Zusammensetzung zur Behandlung allergischer oder entzündlicher Zustände bei einem Säuger, die eine wirksame Menge einer Verbindung der Formel (I) gemäß irgendeinem der vorhergehenden Ansprüche oder ein pharmazeutisch annehmbares Säureadditionssalz derselben und ein pharmazeutisch annehmbares Verdunnungsmittel oder einen solchen Träger umfaßt.

13. Verbindung der Formel (I) gemäß irgendeinem der Ansprüche 1 bis 11 oder ein pharmazeutisch annehmbares Säureadditionssalz derselben zur Verwendung alz Arzneimittel.

14. Die Verwendung einer Verbindung der Formel (I) gemäß irgendeinem der Ansprüche 1 bis 11 oder eines pharmazeutisch annehmbaren Säureadditionssalzes derselben zur Herstellung eines Arzneimittels für die Behandlung allergischer oder entzündlicher Zustände.

15. Verbindung der Formel

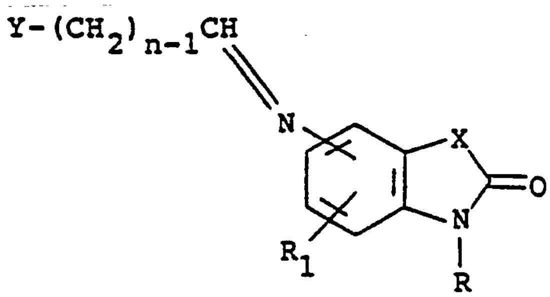

--- (III)

worin R, $R_1$, X, Y und *n* wie in Anspruch 1 definiert sind.

**Patentansprüche für die Vertragsstaaten: AT ES**

1. Verfahren zum Herstellen einer Verbindung mit der Formel

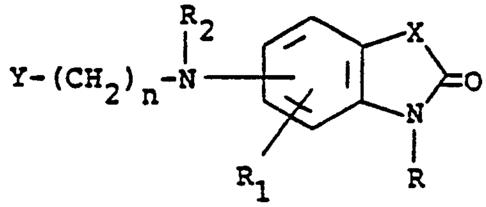

--- (I)

worin R Wasserstoff, Alkyl mit einem bis drei Kohlenstoffatomen, Benzyl oder Carboxymethyl ist, $R_1$ Wasserstoff, Fluor, Chlor, Methoxy, Methyl oder Trifluormethyl ist, $R_2$ Wasserstoff, Alkyl mit einem bis drei Kohlenstoffatomen, Alkanoyl mit einem bis drei Kohlenstoffatomen oder Benzyl ist, X C=O, $CH_2$, NH oder 0 ist, *n* eine ganze Zahl von 0 bis 7 ist und Y Styryl, Methyl, Phenylamino, Phenoxy, Thienyl, Furyl, Pyridyl, Phenyl oder substituiertes Phenyl ist, wobei dieser Substituent Methyl, Methoxy, Fluor oder Chlor ist, unter der Bedingung, daß Y Styryl ist, wenn *n* gleich 0 ist, gekennzeichnet durch Reduzieren einer Verbindung mit der Formel

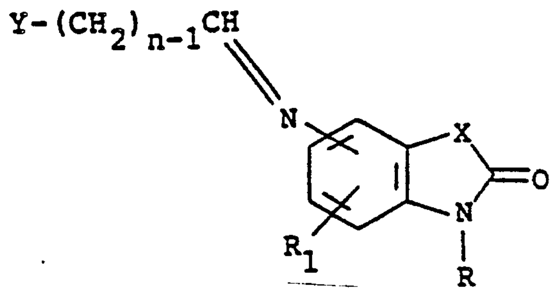

--- (III)

worin Y, *n*, R, $R_1$ und X wie oben definiert sind, falls gewünscht, gefolgt von Alkylieren oder Acylieren einer Verbindung der Formel (I), worin $R_2$ Wasserstoff ist, zur Bildung einer Verbindung der Formel (I), worin $R_2$ Alkyl mit einem bis drei Kohlenstoffatomen, Benzyl oder Alkanoyl mit einem bis drei Kohlenstoffatomen ist, und, nach Wunsch, Umwandeln des Produktes der Formel (I) in ein pharmazeutisch annehmbares Säureadditionssalz durch Reaktion mit einer geeigneten Säure.

2. Verfahren gemäß Anspruch 1, worin das Reduktionsmittel Natriumborhydrid ist.

3. Verfahren gemäß Anspruch 1 oder 2, worin X C=O ist und *n* gleich 3 ist.

4. Verfahren gemäß Anspruch 3, worin Y Phenyl ist und R, $R_1$ und $R_2$ jeweils Wasserstoff sind.

5. Verfahren gemäß Anspruch 1 oder 2, worin X $CH_2$ ist und *n* gleich 1 oder 3 ist.

6. Verfahren gemäß Anspruch 5, worin Y Phenyl ist und R, $R_1$ und $R_2$ jeweils Wasserstoff sind.

7. Verfahren gemäß Anspruch 1 oder 2, worin X NH ist, R Alkyl mit einem bis drei Kohlenstoffatomen ist, $R_1$ Alkoxy mit einem bis drei Kohlenstoffatomen ist und $R_2$ Wasserstoff ist.

8. Verfahren gemäß Anspruch 7, worin *n* gleich 1 ist und Y Styryl ist.

9. Verfahren gemäß Anspruch 7, worin *n* gleich 1 oder 3 ist und Y Phenyl ist.

10. Verfahren gemäß Anspruch 1 oder 2, worin X O ist, Y Styryl, Methyl, Phenyl, Thienyl oder substituiertes Phenyl ist, wobei dieser Substituent Methyl, Methoxy, Fluor oder Chlor ist, R Wasserstoff oder Alkyl mit einem bis drei Kohlenstoffatomen ist, $R_1$ Wasserstoff, Fluor, Chlor, Methoxy oder Methyl ist und $R_2$ Wasserstoff oder Alkyl mit einem bis drei Kohlenstoffatomen ist.

11. Verfahren gemäß Anspruch 10, worin Y Methyl oder Phenyl ist, R und $R_1$ jeweils Wasserstoff sind und *n* gleich 1, 3 oder 5 ist.

12. Verfahren gemäß Anspruch 10, worin Y Phenyl ist, R und $R_2$ jeweils Wasserstoff sind, *n* gleich 1, 3 oder 5 ist und $R_1$ Methyl, Methoxy, Fluor oder Chlor, substituiert in der 5-Position, ist.

13. Verfahren zur Herstellung einer pharmazeutischen Zusammensetzung, gekennzeichnet durch das Mischen einer Verbindung der Formel (I) gemäß Definition in Anspruch 1 oder eines pharmazeutisch annehmbaren Säureadditionssalzes derselben mit einem pharmazeutisch annehmbaren Verdünnungsmittel oder einem solchen Träger.

**Patentansprüche für den Vertragsstaat: GR**

1. Verfahren zum Herstellen einer Verbindung mit der Formel

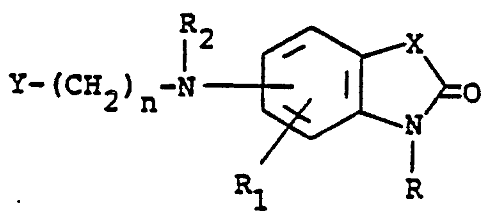

--- (I)

worin R Wasserstoff, Alkyl mit einem bis drei Kohlenstoffatomen, Benzyl oder Carboxymethyl ist, $R_1$ Wasserstoff, Fluor, Chlor, Methoxy, Methyl oder Trifluormethyl ist, $R_2$ Wasserstoff, Alkyl mit einem bis drei Kohlenstoffatomen, Alkanoyl mit einem bis drei Kohlenstoffatomen oder Benzyl ist, X C=O, $CH_2$, NH oder 0 bedeutet, *n* eine ganze Zahl von 0 bis 7 ist und Y Styryl, Methyl, Phenylamino, Phenoxy, Thienyl, Furyl, Pyridyl, Phenyl oder substituiertes Phenyl ist, wobei dieser Substituent Methyl, Methoxy, Fluor oder Chlor ist, unter der Bedingung, daß Y Styryl ist, wenn *n* gleich 0 ist, gekennzeichnet durch Reduzieren einer Verbindung mit der Formel

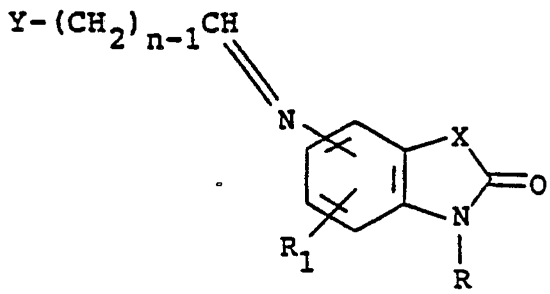

--- (III)

worin Y, *n*, R, $R_1$ und X wie oben definiert sind, falls gewünscht, gefolgt von Alkylieren oder Acylieren einer Verbindung der Formel (I), worin $R_2$ Wasserstoff ist, zur Bildung einer Verbindung der Formel (I), worin $R_2$ Alkyl mit einem bis drei Kohlenstoffatomen, Benzyl oder Alkanoyl mit einem bis drei Kohlenstoffatomen ist, und, nach Wunsch, Umwandeln des Produktes der Formel (I) in ein pharmazeutisch annehmbares Säureadditionssalz durch Reaktion mit einer geeigneten Säure.

2. Verfahren gemäß Anspruch 1, worin das Reduktionsmittel Natriumborhydrid ist.

3. Verfahren gemäß Anspruch 1 oder 2, worin X C=O ist und *n* gleich 3 ist.

4. Verfahren gemäß Anspruch 3, worin Y Phenyl ist und R, $R_1$ und $R_2$ jeweils Wasserstoff sind.

5. Verfahren gemäß Anspruch 1 oder 2, worin X $CH_2$ ist und *n* gleich 1 oder 3 ist.

6. Verfahren gemäß Anspruch 5, worin Y Phenyl ist und R, $R_1$ und $R_2$ jeweils Wasserstoff sind.

7. Verfahren gemäß Anspruch 1 oder 2, worin X NH ist, R Alkyl mit einem bis drei Kohlenstoffatomen ist, $R_1$ Alkoxy mit einem bis drei Kohlenstoffatomen ist und $R_2$ Wasserstoff ist.

8. Verfahren gemäß Anspruch 7, worin *n* gleich 1 ist und Y Styryl ist.

9. Verfahren gemäß Anspruch 7, worin *n* gleich 1 oder 3 ist und Y Phenyl ist.

10. Verfahren gemäß Anspruch 1 oder 2, worin X O ist, Y Styryl, Methyl, Phenyl, Thienyl oder substituiertes Phenyl ist, wobei dieser Substituent Methyl, Methoxy, Fluor oder Chlor ist, R Wasserstoff oder Alkyl mit einem bis drei Kohlenstoffatomen ist, $R_1$ Wasserstoff, Fluor, Chlor, Methoxy oder Methyl ist und $R_2$ Wasserstoff oder Alkyl mit einem bis drei Kohlenstoffatomen ist.

11. Verfahren gemäß Anspruch 10, worin Y Methyl oder Phenyl ist, R und $R_1$ jeweils Wasserstoff sind und *n* gleich 1, 3 oder 5 ist.

12. Verfahren gemäß Anspruch 10, worin Y Phenyl ist, R und $R_2$ jeweils Wasserstoff sind, *n* gleich 1, 3 oder 5 ist und $R_1$ Methyl, Methoxy, Fluor oder Chlor, substituiert in der 5-Position, ist.

13. Verfahren zur Herstellung einer pharmazeutischen Zusammensetzung, gekennzeichnet durch das Mischen einer Verbindung der Formel (I) gemäß Definition in Anspruch 1 oder eines pharmazeutisch annehmbaren Säureadditionssalzes derselben mit einem pharmazeutisch annehmbaren Verdünnungsmittel oder einem solchen Träger.

14. Verbindung der Formel:

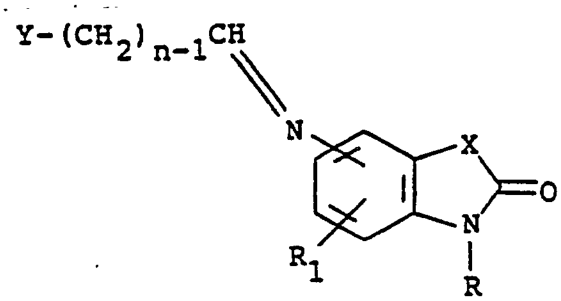

--- (III)

worin Y, *n*, $R_1$ und X wie in Anspruch 1 definiert sind.

**Revendications pour les Etats contractants: BE CH DE FR GB IT LI LU NL SE**

1. Composé de formule:

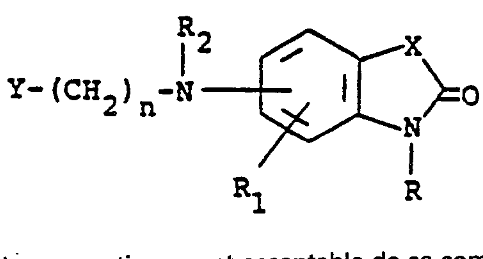

--- (I)

ou un sel d'addition d'acide pharmaceutiquement acceptable de ce composé, formule dans laquelle R est l'hydrogène, un groupe alkyle ayant un à trois atomes de carbone, benzyle ou carboxyméthyle; $R_1$ est l'hydrogène, un radical fluoro, chloro, méthoxy, méthyle ou trifluorométhyle; $R_2$ est l'hydrogène, un groupe alkyle ayant un à trois atomes de carbone, alcanoyle ayant un à trois atomes de carbone, ou benzyle; X représente C=O, $CH_2$, NH ou O; *n* est un nombre entier de 1 à 7; et Y est un groupe styryle, méthyle, phénylamino, phénoxy, thiényle, furyle, pyridyle, phényle ou phényle substitué dont le substituant est un radical méthyle, méthoxy, fluoro ou chloro.

2. Composé suivant la revendication 1, dans lequel X est un groupe C=O et *n* a la valeur 3.

3. Composé suivant la revendication 2, dans lequel Y est un groupe phényle et R, $R_1$ et $R_2$ sont chacun de l'hydrogène.

4. Composé suivant la revendication 1, dans lequel X est un groupe $CH_2$ et *n* a la valeur 1 ou 3.

5. Composé suivant la revendication 4, dans lequel Y est un groupe phényle et R, $R_1$ et $R_2$ sont chacun de l'hydrogène.

6. Composé suivant la revendication 1, dans lequel X est un groupe NH, R est un groupe alkyle ayant un à trois atomes de carbone, $R_1$ est un groupe alkoxy ayant un à trois atomes de carbone et $R_2$ est l'hydrogène.

7. Composé suivant la revendication 6, dans lequel *n* a la valeur 1 et Y est un groupe styryle.

8. Composé suivant la revendication 6, dans lequel *n* a la valeur 1 ou 3 et Y est un groupe phényle.

9. Composé suivant la revendication 1, dans lequel X représente O, Y est un groupe styryle, méthyle, phényle, thiényle ou phényle substitué dont le substituant est un radical méthyle, méthoxy, fluoro ou chloro; R est l'hydrogène ou un groupe alkyle ayant un à trois atomes de carbone; $R_1$ est l'hydrogène ou un radical fluoro, chloro, méthoxy ou méthyle; et $R_2$ est l'hydrogène ou un groupe alkyle ayant un à trois atomes de carbone.

10. Composé suivant la revendication 9, dans lequel Y est un groupe méthyle ou phényle, R et $R_1$ représentent chacun l'hydrogène et *n* a la valeur 1, 3 ou 5.

11. Composé suivant la revendication 9, dans lequel Y est un groupe phényle, R et $R_2$ sont tous deux de l'hydrogène, *n* a la valeur 1, 3 ou 5 et $R_1$ est un substituant méthyle, méthoxy, fluoro ou chloro attaché en position 5.

12. Composition pharmaceutique destinée au traitement de conditions allergiques ou inflammatoires chez un mammifère, ladite composition comprenant une quantité efficace d'un composé de formule (I) suivant l'une quelconque des revendications précédentes ou d'un sel d'addition d'acide pharmaceutiquement acceptable de ce composé, et un diluant ou support acceptable du point de vue pharmaceutique.

13. Composé de formule (I) suivant l'une quelconque des revendications 1 à 11, ou sel d'addition d'acide pharmaceutiquement acceptable de ce composé, destiné à être utilisé comme médicament.

14. Utilisation d'un composé de formule (I) suivant l'une quelconque des revendications 1 à 11 ou d'un sel d'addition d'acide pharmaceutiquement acceptable de ce composé pour la préparation d'un médicament destiné au traitement de conditions allergiques ou inflammatoires.

15. Composé de formule:

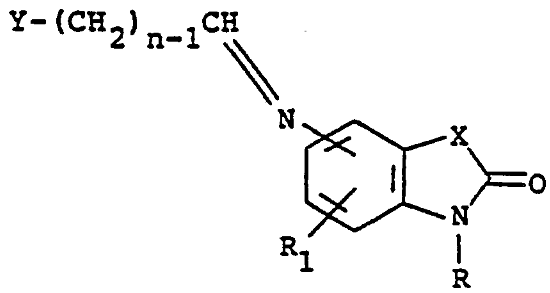

--- (III)

dans laquelle R, $R_1$, X, Y et n sont tels que définis dans la revendication 1.

**Revendications pour les Etats contractants: AT ES**

1. Procédé de préparation d'un composé de formule:

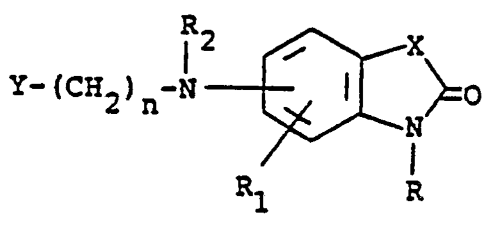

--- (I)

ou d'un sel d'addition d'acide pharmaceutiquement acceptable de ce composé, formule dans laquelle R est l'hydrogène, un groupe alkyle ayant un à trois atomes de carbone, benzyle ou carboxyméthyle; $R_1$ est l'hydrogène, un radical fluoro, chloro, méthoxy, méthyle ou trifluorométhyle; $R_2$ est l'hydrogène, un groupe alkyle ayant un à trois atomes de carbone, alcanoyle ayant un à trois atomes de carbone, ou benzyle; X représente C=O, $CH_2$, NH ou O; *n* est un nombre entier de 1 à 7; et Y est un groupe styryle, méthyle, phénylamino, phénoxy, thiényle, furyle, pyridyle, phényle ou phényle substitué dont le substituant est un radical méthyle, méthoxy, fluoro ou chloro, caractérisé par la réduction d'un composé de formule:

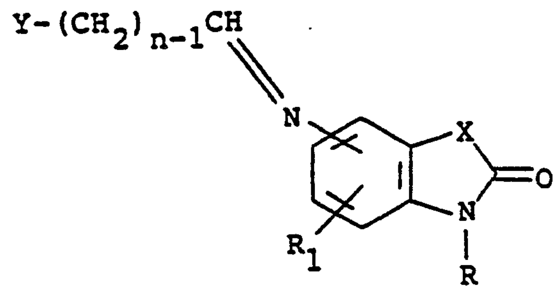

--- (III)

dans laquelle Y, n, R, $R_1$ et X sont tels que définis ci-dessus, suivie, le cas échéant, d'une alkylation ou d'une acylation d'un composé de formule (I) dans laquelle $R_2$ est l'hydrogène de manière à produire un composé de formule (I) dans laquelle $R_2$ est un groupe alkyle ayant 1 à 3 atomes de carbone, benzyle ou alcanoyle ayant 1 à 3 atomes de carbone et, le cas échéant, transformation du produit de formule (I) en un sel d'addition d'acide pharmaceutiquement acceptable par réaction avec un acide convenable.

2. Procédé suivant la revendication 1, dans lequel l'agent réducteur est le borohydrure de sodium.

3. Procédé suivant la revendication 1 ou 2, dans lequel X représente C=O et *n* a la valeur 3.

4. Procédé suivant la revendication 3, dans lequel Y est un groupe phényle et R, $R_1$ et $R_2$ représentent chacun l'hydrogène.

5. Procédé suivant la revendication 1 ou 2, dans lequel X est un groupe $CH_2$ et *n* a la valeur 1 ou 3.

6. Procédé suivant la revendication 5, dans lequel Y est un groupe phényle et R, $R_1$ et $R_2$ représentent chacun l'hydrogène.

7. Procédé suivant la revendication 1 ou 2, dans lequel X représente NH, R est un groupe alkyle ayant un à trois atomes de carbone, $R_1$ est un groupe alkoxy ayant un à trois atomes de carbone et $R_2$ est l'hydrogène.

8. Procédé suivant la revendication 7, dans lequel *n* à 1 et Y est un groupe styryle.

9. Procédé suivant la revendication 7, dans lequel *n* a la valeur 1 ou 3 et Y est un groupe phényle.

10. Procédé suivant la revendication 1 ou 2, dans lequel X représente O, Y est un groupe styryle, méthyle, phényle, thiényle ou phényle substitué dont le substituant est un radical méthyle, méthoxy, fluoro ou chloro; R est l'hydrogène ou un groupe alkyle ayant un à trois atomes de carbone; $R_1$ est l'hydrogène ou un radical fluoro, chloro, méthoxy ou méthyle; et $R_2$ est l'hydrogène ou un groupe alkyle ayant un à trois atomes de carbone.

11. Procédé suivant la revendication 10, dans lequel Y est un groupe méthyle ou phényle, R et $R_1$ sont tous deux de l'hydrogène et *n* a la valeur 1, 3 ou 5.

12. Procédé suivant la revendication 10, dans lequel Y est un groupe phényle, R et $R_2$ sont tous deux de l'hydrogène, *n* a la valeur 1, 3 ou 5 et $R_1$ est un substituant méthyle, méthoxy, fluoro ou chloro en position 5.

13. Procédé de préparation d'une composition pharmaceutique, caractérisé par le mélange d'un composé de formule (I) tel que défini dans la revendication 1, ou d'un sel d'addition d'acide pharmaceutiquement acceptable de ce composé, avec un diluant ou support acceptable du point de vue pharmaceutique.

**Revendications pour l'Etat contractant: GR**

1. Procédé de préparation d'un composé de formule:

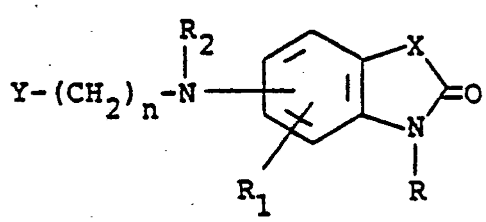

--- (I)

ou d'un sel d'addition d'acide pharmaceutiquement acceptable de ce composé, formule dans laquelle R est l'hydrogène, un groupe alkyle ayant un à trois atomes de carbone, benzyle ou carboxyméthyle; $R_1$ est l'hydrogène, un radical fluoro, chloro, méthoxy, méthyle ou trifluorométhyle; $R_2$ est l'hydrogène, un groupe alkyle ayant un à trois atomes de carbone, alcanoyle ayant un à trois atomes de carbone, ou benzyle; X représente C=O, $CH_2$, NH ou O; *n* est un nombre entier de 1 à 7; et Y est un groupe styryle, méthyle, phénylamino, phénoxy, thiényle, furyle, pyridyle, phényle ou phényle substitué dont le substituant est un radical méthyle, méthoxy, fluoro ou chloro, caractérisé par la réduction d'un composé de formule:

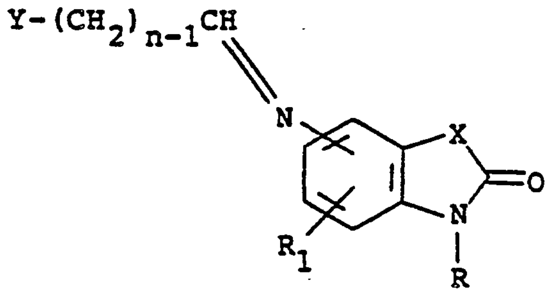

--- (III)

dans laquelle Y, n, R, $R_1$ et X sont tels que définis ci-dessus, suivie, le cas échéant, d'une alkylation ou d'une acylation d'un composé de formule (I) dans laquelle $R_2$ est l'hydrogène de manière à produire un composé de formule (I) dans laquelle $R_2$ est un groupe alkyle ayant 1 à 3 atomes de carbone, benzyle ou alcanoyle ayant 1 à 3 atomes de carbone et, le cas échéant, transformation du produit de formule (I) en un sel d'addition d'acide pharmaceutiquement acceptable par réaction avec un acide convenable.

2. Procédé suivant la revendication 1, dans lequel l'agent réducteur est le borohydrure de sodium.

3. Procédé suivant la revendication 1 ou 2, dans lequel X représente C=O et *n* a la valeur 3.

4. Procédé suivant la revendication 3, dans lequel Y est un groupe phényle et R, $R_1$ et $R_2$ représentent chacun l'hydrogène.

5. Procédé suivant la revendication 1 ou 2, dans lequel X est un groupe $CH_2$ et *n* a la valeur 1 ou 3.

6. Procédé suivant la revendication 5, dans lequel Y représentent phényle et R, $R_1$ et $R_2$ représentent chacun l'hydrogène.

7. Procédé suivant la revendication 1 ou 2, dans lequel X représentent NH, R est un groupe alkyle ayant un à trois atomes de carbone, $R_1$ est un groupe alkoxy ayant un à trois atomes de carbone et $R_2$ est l'hydrogène.

8. Procédé suivant la revendication 7, dans lequel *n* est egal à 1 et Y est un groupe styryle.

9. Procédé suivant la revendication 7, dans lequel *n* a la valeur 1 ou 3 et Y est un groupe phényle.

10. Procédé suivant la revendication 1 ou 2, dans lequel X représente O, Y est un groupe styryle, méthyle, phényle, thiényle ou phényle substitué dont le substituant est un radical méthyle, méthoxy, fluoro ou chloro; R est l'hydrogène ou un groupe alkyle ayant un à trois atomes de carbone; $R_1$ est l'hydrogène ou un radical fluoro, chloro, méthoxy ou méthyle; et $R_2$ est l'hydrogène ou un groupe alkyle ayant un à trois atomes de carbone.

11. Procédé suivant la revendication 10, dans lequel Y est un groupe méthyle ou phényle, R et $R_1$ sont tous deux de l'hydrogène et *n* a la valeur 1, 3 ou 5.

12. Procédé suivant la revendication 10, dans lequel Y est un groupe phényle, R et $R_2$ sont tous deux de l'hydrogène, *n* a la valeur 1, 3 ou 5 et $R_1$ est un substituant méthyle, méthoxy, fluoro ou chloro en position 5.

13. Procédé de préparation d'une composition pharmaceutique, caractérisé par le mélange d'un composé de formule (I) tel que défini dans la revendication 1, ou d'un sel d'addition d'acide pharmaceutiquement acceptable de ce composé, avec un diluant ou support acceptable du point de vue pharmaceutique.

14. Composé de formule:

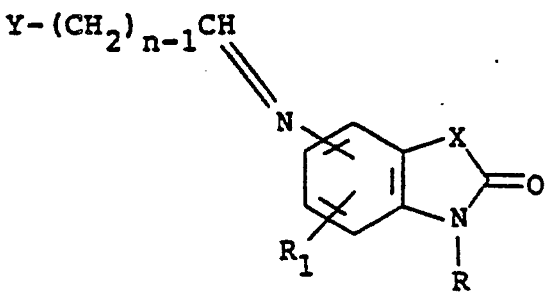

--- (III)

dans laquelle Y, n, R, $R_1$, et X sont tels que définis dans la revendication 1.